## Europäisches Patentamt
## European Patent Office
## Office européen des brevets

(19)

(11) Veröffentlichungsnummer: **0 008 728**
**B1**

(12)

# EUROPÄISCHE PATENTSCHRIFT

(45) Veröffentlichungstag der Patentschrift:
23.03.83

(51) Int. Cl.³: **C 12 N 1/06**

(21) Anmeldenummer: **79103036.4**

(22) Anmeldetag: **20.08.79**

(54) Verfahren zur Herstellung von Hefeautolysat.

(30) Priorität: **26.08.78 DE 2837342**

(43) Veröffentlichungstag der Anmeldung:
**19.03.80 Patentblatt 80/6**

(45) Bekanntmachung des Hinweises auf die Patenterteilung:
**23.03.83 Patentblatt 83/12**

(84) Benannte Vertragsstaaten:
**DE FR GB NL**

(56) Entgegenhaltungen:
**DE-C-441 457**
**FR-A-533 580**
**FR-A-599 977**
**FR-A-899 094**
**GB-A-1 432 039**
**GB-A-2 009 785**
**US-A-2 368 384**
**US-A-2 450 604**

(73) Patentinhaber: **Deutsche Hefewerke GmbH,
Wandsbeker Zollstrasse 59, D-2000 Hamburg-Wandsbek
(DE)**

(72) Erfinder: **Hill, Frank Friedrich, Dr., Schlesienstrasse 23,
D-4020 Mettmann-Obschwarzbach (DE)**

## Verfahren zur Herstellung von Hefeautolysat

Die Erfindung betrifft ein Verfahren zur Herstellung von Hefeautolysat.

Extrakte aus Hefen sind ursprünglich als Ersatzstoffe für Fleischextrakt zur Aromatisierung von Nahrungsmitteln und Haustierfutter verwendet worden. Die Bedeutung der Hefeextrakte als natürlicher Aromastoff hat im Verlauf der letzten Jahrzehnte zugenommen. Technisch können Extrakte aus Hefen auf verschiedene Weise hergestellt werden. Die Verfahren unterscheiden sich sowohl in ihrer prinzipiellen Vorgehensweise als auch in den funktionellen Eigenschaften der erhaltenen Produkte. Die drei Varianten zur Extraktion der Hefe sind Plasmolyse, Autolyse und Hydrolyse. Die Gewinnung von Hefeextrakten mittels Autolyse hat die größte technische Bedeutung. Die Herstellung von Hefeextrakten mittels Autolyse — die im folgenden als Hefeautolysate bezeichnet werden — ist beschrieben in dem Handbuch »Die Hefen«, Bd. II, S. 761 – 764 (Verlag H. Carl, Nürnberg 1962) sowie in der Zeitschrift Process Biochemistry 1, 313 – 317 (1966) und 5, 50 – 52 (1970).

Zur Herstellung von Hefeautolysaten eignen sich verschiedene Gattungen der Hefen, die auf verschiedenen Kohlenstoffquellen herangezüchtet werden. Im technischen Maßstab werden schnellwachsende Hefen der Gattungen Saccharomyces, Candida und Torula auf billigen Kohlenstoffquellen wie Melasse, Malzextrakt, Molke, Sulfitablauge, Äthanol und/oder Alkanen kultiviert. Der Nährwert der lebenden Hefen ist gering. Durch die Abtrennung der Hefezellwände sowie den hydrolytischen Abbau der Zellinhaltsstoffe haben die Hefeautolysate höhere Nährwerte und bessere Aromaeigenschaften. Bei der Gewinnung von Hefeextrakt mittels Autolyse kommt es darauf an, den hydrolytischen Abbau der Zellinhaltsstoffe durch zelleigene Enzyme zu starten ohne durch den notwendigen Impuls, der die lebende Hefe zur Selbstverdauung veranlaßt, die hydrolytisch aktiven Enzyme zu zerstören. Der Impuls kann durch Zerstören der Zellstrukturen mittels Zermahlen oder Zerplatzen gegeben werden. Geeignete Apparate für diese Verfahrensschritte sind bekannt, sie benötigen jedoch einen zusätzlichen, hohen Energieaufwand. Durch die Trocknung der Hefe vor der Autolyse werden die Zellmembranen labil und die Autolyse der resuspendierten Hefe wird begünstigt (DE-OS 2 359 501). Am häufigsten werden organische Lösungsmittel zur Labilisierung der Zellmembranen und Begünstigung der Autolyse verwendet (»Die Hefen«, Bd. II, S. 761 – 764). Bevorzugt werden Lösungsmittel wie Äthanol, Äthylacetat, Toluol und chlorierte Kohlenwasserstoffe eingesetzt. In neueren Verfahren wird die Lysis der Hefezelle durch zugesetzte Enzyme aus verschiedenen Mikroorganismen eingeleitet.

In der Patentschrift FR-899 094 wird ein Verfahren zur Plasmolyse oder Autolyse von Hefe beschrieben, bei dem man das Medium mit Bakterien, speziell mit Lacto- und Butyrobakterien, impft und unter für die Bakterien günstigen Bedingungen bebrütet. Die Bakterien erzeugen Säuren, die durch Zusatz von Ammoniumcarbonat schrittweise neutralisiert werden; Säurebildung und Neutralisation werden mehrfach wiederholt. Nach der Behandlung der Hefe mit Bakterien wird sie bei erhöhter Temperatur plasmolysiert oder autolysiert.

Ziel der Erfindung ist es, den Einsatz von aufwendigen mechanischen Vorrichtungen bzw. teuren oder toxischen Hilfsstoffen zu vermeiden.

Gegenstand der Erfindung ist ein Verfahren zur Herstellung von Hefeautolysat, dadurch gekennzeichnet, daß man die Autolyse in wäßriger Suspension unter Zusatz von 0,03 – 15 Gew.-%, vorzugsweise 0,3 – 6,0 Gew.-%, bezogen auf Hefe-Trockenmasse, an Fettsäuren der Kettenlängen $C_4 - C_{14}$ und/oder deren Glyzerinester bei einer Temperatur von 20 – 60° C und einer Zeitdauer von 6 – 36 Stunden durchführt, die Hefeschalen von der Flüssigkeit abtrennt und das Autolysat durch Eindampfen gewinnt.

Vorzugsweise wird die Autolyse unter Zusatz von 0,1 – 10 Gew.-% Kochsalz zur Hefesuspension durchgeführt. Die Autolyse kann beschleunigt bzw. die Menge an einzusetzender Fettsäure bzw. Fettsäureglyzerinester reduziert werden, wenn man die Hefe vor der Autolyse einem Temperaturschock von 60 – 90° C während einer Zeitdauer von 1 – 120 Sekunden aussetzt. Hierzu wird die Hefesuspension beispielsweise durch eine Spirale aus Kupferrohr gepumpt, die in ein erhitztes Wasserbad eingehängt ist, wobei die Zeitdauer des Durchpumpens und die Temperatur des Wasserbades für die Temperaturschock-Wirkung maßgebend sind.

Die Anwendung des Temperaturschock-Verfahrens hat den besonderen Vorteil, daß hierdurch die benötigte Fettsäuremenge bis auf unter 0,2 Gew.-% der Hefe-Trockenmasse herabgesetzt werden kann. Durch die geringe, dann noch im Endprodukt verbleibende Menge an Fettsäure wird der charakteristische Geschmack des herkömmlichen Hefeautolysates nicht beeinträchtigt.

Für die Autolyse wird eine Hefesuspension mit einem Gehalt von 10 – 20 Gew.-% Hefe-Trockenmasse eingesetzt. Die Autolyse selbst erfolgt nach Zusatz der erforderlichen Menge an Fettsäure oder Fettsäureglyzerinester unter Rühren der Hefesuspension bei einer Temperatur von 20 – 60° C, vorzugsweise 30 – 60° C, während einer Zeitdauer von 6 – 36 Stunden. Es werden vorzugsweise 0,3 – 6,0 Gew.-% an Fettsäure bzw. Fettsäureglyzerinester, bezogen auf Hefe-Trockenmasse, benötigt, um ein gutes Ergebnis zu erzielen. Bei Anwendung eines Temperaturschocks kann die Fettsäure- bzw. Fettsäureglyzerinester-Menge bis auf 0,03% der Hefe-Trockenmasse herabgesetzt werden.

Die Gewinnung des Autolysates erfolgt durch Abtrennen der Hefeschalen, z. B. durch Filtrieren-oder Zentrifugieren und Eindampfen der wäßrigen, das Autolysat enthaltenden Lösung. Es empfiehlt sich, die abgetrennten Hefeschalen mehrmals mit Wasser oder Kochsalzlösung nachzuwaschen und die Waschwässer mit der Hauptmenge der abgetrennten wäßrigen Lösung zu vereinigen. Die Ausbeuten an Autolysat liegen in der Regel oberhalb von 30 Gew.-%, vorzugsweise zwischen 40 und 65 Gew.-%, bezogen auf eingesetzte Hefe-Trockenmasse.

Das Verfahren wird unter Zusatz von Fettsäuren der Kettenlängen $C_4 - C_{14}$ durchgeführt. Bevorzugt werden Capryl- und Caprinsäure bzw. deren Gemische eingesetzt, doch werden auch mit Buttersäure, Capronsäure, Laurinsäure und Myristinsäure günstige Ergebnisse erzielt. In gleicher Weise wie die freien Säuren können auch deren Mono-, Di- oder Triglyzeride verwendet werden, z. B. Caprinsäuremonoglyzerid oder Caprylsäuretriglyzerid bzw. deren Mischungen oder Mischester. Im allgemeinen werden mit den freien Säuren sowie mit den Mono- und Diglyzeriden die günstigeren Ergebnisse erzielt. Da die Fettsäuren und ihre Glycerinester eßbar sind, können sie vollständig oder weitgehend im Autolysat verbleiben.

Geeignete Hefearten, die dem Autolyseverfahren unterworfen werden, sind Candida utilis, Saccharomyces cerevisiae, Saccharomyces fragilis und Saccharomyces carlsbergensis.

### Beispiel 1

25 g einer handelsüblichen Preßhefe (Saccharomyces cerevisiae) mit 30% Trockensubstanz werden in Wasser suspendiert und auf 50 ml aufgefüllt. Zu dem Ansatz werden je 200 mg Kochsalz und 75 mg des in Tabelle 1 aufgeführten Zusatzstoffes gegeben. Der Ansatz wird in einem Wasserbad bei 50° C mit 150 UpM geschüttelt. Nach 16 Stunden werden 2 Proben von 5 ml entnommen. Probe 1 wird ohne weitere Behandlung in einem Aluminiumschälchen bei 110° C in einem Trockenschrank eingedampft und getrocknet. Probe 2 wird zentrifugiert und das Sediment 2× mit 5 ml destilliertem Wasser gewaschen. Die vereinigten Überstände werden ebenfalls bei 110° C eingedampft und getrocknet. Die in Tabelle 1 wiedergegebenen Trockengewichte zeigen die ausgezeichnete stimulierende Wirkung von Caprinsäure bzw. Caprin-/Caprylsäuremonoglyzerid auf die Hefeautolyse im Vergleich zu verschiedenen organischen Lösungsmitteln.

Tabelle 1

| Zusatzstoff (75 mg/25 g Hefe = 0,3 Gew.-%) | Probe 1 Trockengewicht Hefeschalen + Autolysat | Probe 2 Autolysat | |
|---|---|---|---|
| | (mg) | (mg) | (Gew.-%) |
| — | 837.2 | 224.0 | 26.8 |
| Äthylacetat | 867.6 | 248.3 | 28.7 |
| Toluol | 897.4 | 330.8 | 37.0 |
| Tetrachlorkohlenstoff | 856.7 | 326.6 | 38.1 |
| Trichloräthylen | 845.0 | 328.0 | 38.8 |
| Caprinsäure | 880.6 | 448.5 | 50.9 |
| Caprin-/Caprylsäure-1 : 1-mono-glyzerid | 866.0 | 480.5 | 55.5 |

### Beispiel 2

In einem wie in Beispiel 1 durchgeführten Ansatz wird die Wirkung verschiedener natürlicher Fettsäuren auf die Hefeautolyse geprüft. Es zeigt sich (Tab. 2), daß Fettsäuren mit einer Anzahl von 4 – 14 Kohlenstoffatomen besonders geeignet sind.

Tabelle 2

| Zusatzstoff (75 mg/25 g Hefe = 0,3 Gew.-%) | Probe 1 Trockengewicht Hefeschalen + Autolysat | Probe 2 Autolysat | |
|---|---|---|---|
| | (mg) | (mg) | (Gew.-%) |
| — | 837.2 | 224.0 | 26.8 |
| Propionsäure | 845.2 | 310.2 | 36.7 |
| Buttersäure | 844.0 | 370.5 | 43.9 |
| Valeriansäure | 904.4 | 433.3 | 47.9 |
| Caprylsäure | 874.2 | 451.1 | 51.7 |
| Caprinsäure | 867.4 | 426.8 | 49.2 |
| Laurinsäure | 843.5 | 411.5 | 48.8 |
| Myristinsäure | 860.4 | 395.0 | 45.8 |
| Palmitinsäure | 825.4 | 222.7 | 27.0 |

### Beispiel 3

Die Menge der für die Autolysebeschleunigung benötigten Fettsäure kann durch eine thermische Schockbehandlung der Hefe deutlich reduziert werden. Dazu werden 2,5 kg Preßhefe (30% Trockensubstanz) in Wasser suspendiert, mit 100 g Kochsalz versetzt und auf 5,0 l aufgefüllt. Die Suspension wird durch eine Spirale aus Kupferrohr gepumpt, die in ein auf 70°C thermostatisiertes Wasserbad gehängt wird. Die Länge der Spirale und die Geschwindigkeit der Suspensionsförderung wurden auf eine Verweilzeit der Hefesuspension im erwärmten Wasserbad von 10 sec eingestellt. Nach dem Durchgang durch das Metallrohr wurde die Hefesuspension in einem weiteren Wasserbad auf 40°C abgekühlt und dann in einem 10-l-Glasbehälter aufgefangen und bei 50°C gerührt. Der Ansatz war nach ca. 60 min vollständig durch die kontinuierliche Hitzeschockbehandlung gepumpt. Anschließend wurde der Ansatz mit 0,1 Gew.-% Caprinsäure, bezogen auf das Gewicht der Preßhefe, versetzt und 16 Stunden bei 50°C gerührt. Die Probenaufarbeitung wurde wie in Beispiel 1 angegeben, durchgeführt. Zusätzlich wurde der Geschmack der erhaltenen Endprodukte geprüft. Dazu wurde nach dem Abzentrifugieren der Hefezellwände aus dem Autolyseansatz der Überstand im Wasserstrahlvakuum bei 60°C eingedampft (auf ca. 70% Trockensubstanz). Aus dem dickflüssigen Autolysat wurde eine 2%ige Lösung mit destilliertem Wasser bereitet, die auf 60°C erwärmt und verkostet wurde. Beide Proben schmeckten sehr ähnlich und unterschieden sich lediglich in Geschmacksnuancen. Ein störender Fettsäuregeschmack war nicht festzustellen.

Tabelle 3

| Zusatzstoff (0,1 Gew.-% auf Preßhefe) | Probenahme aus dem Autolyse- ansatz nach Inkubationszeit | Probe 1 Trockengewicht Hefeschalen Autolysat | Probe 2 Autolysat | |
|---|---|---|---|---|
| | (h) | (mg) | (mg) | (Gew.-%) |
| — | 1 | 952.4 | 264.2 | 27.8 |
| | 16 | 971.2 | 455.7 | 46.9 |
| Caprinsäure | 2 | 944.5 | 411.3 | 43.5 |
| | 18 | 979.0 | 606.3 | 61.9 |

**Patentansprüche**

1. Verfahren zur Herstellung von Hefeautolysat, dadurch gekennzeichnet, daß man die Autolyse in wäßriger Suspension unter Zusatz von 0,03 bis 15 Gew.-%, vorzugsweise 0,3 bis 6 Gew.-%, bezogen auf Hefe-Trockenmasse, an Fettsäuren der Kettenlängen $C_4$ bis $C_{14}$ und/oder deren Glyzerinester bei einer Temperatur von 20 bis 60°C und einer Zeitdauer von 6 bis 36 Stunden durchführt, die Hefeschalen von der Flüssigkeit abtrennt und das Autolysat durch Eindampfen gewinnt.

2. Verfahren nach Anspruch 1, dadurch gekennzeichnet, daß man die wäßrige Hefesuspension vor der Autolyse einem Temperaturschock von 60 bis 90°C während einer Zeitdauer von 1 bis 120 Sekunden aussetzt.

3. Verfahren nach Anspruch 1 und 2, dadurch gekennzeichnet, daß man die Autolyse unter Zusatz von Capryl- und/oder Caprinsäure durchführt.

4. Verfahren nach Anspruch 1 bis 3, dadurch gekennzeichnet, daß man die Autolyse unter Zusatz von Mono- und/oder Diglyzeriden der Capryl- und/oder Caprinsäure durchführt.

**Claims**

1. A process for producing a yeast autolysate, characterised in that the autolysis is carried out in aqueous suspension at a temperature of 20 to 60°C for 6 to 36 hours with the addition of 0.03 to 15% by weight, preferably 0.3 to 6% by weight, based on the dry yeast weight, of one or more fatty acids having a $C_4$ to $C_{14}$ carbon chain and/or their glycerides, the yeast husks are separated from the liquor and the autolysate is recovered by evaporation.

2. A process according to claim 1, characterised in that the aqueous yeast suspension is exposed to a temperature »shock« of 60 to 90°C for 1 to 120 seconds before the autolysis.

3. A process according to claim 1 or 2, characterised in that the autolysis is carried out with the addition of caprylic acid and/or capric acid.

4. A process according to any of claims 1 to 3, characterised in that the autolysis is carried out with the addition of a monoglyceride and/or a diglyceride of caprylic acid and/or of capric acid.

**Revendications**

1. Procédé de préparation d'un autolysat de levure, caractérisé par le fait qu'on effectue l'autolyse en suspension aqueuse en ajoutant de 0,03 à 15% en poids, de préférence de 0,3 à 6% en poids, relativement à la masse sèche de levure, d'acides gras d'une longueur de chaîne de 4 à 14 atomes de carbone et/ou de leurs esters de glycérol, à une température de 20 à 60°C et pendant une période de temps de 6 à 36 heures, qu'on sépare les membranes de levure du liquide et qu'on obtient l'autolysat par évaporation.

2. Procédé selon la revendication 1, caractérisé par le fait qu'on soumet la suspension aqueuse de levure, avant l'autolyse, à un choc thermique de 60 à 90°C pendant une période de temps de 1 à 120 secondes.

3. Procédé selon les revendications 1 et 2, caractérisé par le fait qu'on effectue l'autolyse en ajoutant de l'acide caprylique et/ou de l'acide caprique.

4. Procédé selon les revendications 1 à 3, caractérisé par le fait qu'on effectue l'autolyse en ajoutant des mono- et/ou di-glycérides de l'acide caprylique et/ou de l'acide caprique.